# EUROPEAN PATENT SPECIFICATION

(11) **EP 2 854 890 B1**
(45) Date of publication and mention of the grant of the patent: **06.02.2019**
(21) Application number: 13729833.7
(22) Date of filing: 21.05.2013
(51) Int. Cl.: A61L 31/06, A61L 31/16

(54) **INJECTABLE BIODEGRADABLE PARTICLES FOR CONTROLLED THERAPEUTIC AGENT RELEASE**
INJIZIERBARE BIOLOGISCH ABBAUBARE PARTIKEL ZUR GESTEUERTEN WIRKSTOFFFREISETZUNG
PARTICULES BIODÉGRADABLES INJECTABLES POUR LIBÉRATION CONTRÔLÉE D'AGENT THÉRAPEUTIQUE

(30) Priority: 30.05.2012 US 201261653233 P
(43) Date of publication of application: 08.04.2015
(73) Proprietor: Boston Scientific Scimed, Inc., Maple Grove, MN 55311-1566 (US)
(72) Inventor: WEBER, Jan, 6228 GJ Maastricht (NL)
(74) Representative: Vossius & Partner Patentanwälte Rechtsanwälte mbB
(86) International application number: PCT/US2013/042025
(87) International publication number: WO 2013/181022

(56) References cited:
- WO-A1-2005/058198
- WO-A2-2005/051234
- US-A1- 2004 063 606
- US-A1- 2005 129 775
- US-A1- 2009 311 304

## Description

### FIELD OF THE INVENTION

The invention relates to polymeric particles for injection which exhibit controlled therapeutic agent release.

### BACKGROUND OF THE INVENTION

Many clinical situations benefit from regulation of the vascular, lymphatic or duct systems by restricting the flow of body fluid or secretions. For example, the technique of embolization involves the introduction of particles into the circulation to occlude blood vessels, for example, so as to either arrest or prevent hemorrhaging or to cut off blood flow to a structure or organ. Temporary occlusion of blood vessels is desirable for managing various diseases and conditions.

In one example of an embolization procedure, local anesthesia is first given over a common artery. The artery is then percutaneously punctured and a catheter is inserted and fluoroscopically guided into the area of interest. An angiogram is then performed by injecting contrast agent through the catheter. An embolic agent is then deposited through the catheter. The embolic agent is chosen, for example, based on the size of the vessel to be occluded, the desired duration of occlusion, and/or the type of disease or condition to be treated (e.g., hypervascular tumors, uterine fibroids, etc.), among others factors. A follow-up angiogram may be performed to determine the specificity and completeness of the arterial occlusion. Blocking the blood supply to the tissue is intended to result in shrinkage and/or death of the tissue.

Various microspheres are currently employed to embolize blood vessels. These microspheres are usually introduced to the location of the intended embolization through microcatheters. Many commercially available embolic microspheres are composed of polymers. Materials used commercially for this purpose include polyvinyl alcohol (PVA), acetalized PVA (e.g., Contour SE™ embolic agent, Boston Scientific, Natick, MA, USA) and crosslinked acrylic hydrogels (e.g., Embospheres®, Biosphere Medical, Rockland, MA, USA). Similar microspheres have been used in chemoembolization to increase the residence time of the therapeutic after delivery. Other examples of commercially available microspheres include glass microspheres with entrapped radioisotopes (e.g., ⁹⁰Y), in particular, TheraSpheres™, MDS Nordion, Ottowa, Canada and polymer microspheres that are capable of chelating radioisotopes (⁹⁰Y), in particular, SIR-Spheres®, SIRTex Medical, New South Wales, Australia. In one specific instance, a therapeutic agent (doxorubicin) has been directly added to polyvinyl alcohol hydrogel microspheres such that it can be released locally after delivery (e.g., DC Bcad™ drug delivery chemoembolization system, Biocompatibles International plc, Farnham, Surrey, UK). There are also particles currently on the market (e.g. Embosphere®, Merit Medical Systems, Inc., South Jordan, Utah USA) that allow the health care provider to load "empty" porous beads with a drug. Loading is typically achieved by ionic bonding of the drug to the particle, resulting in a relatively low drug uptake by the particles and a relatively fast release (e.g., within 48 hours). WO 2005/051234 A2 discloses an injectable drug delivery device which comprises a core containing one or more drugs and one or more polymers.

### SUMMARY OF THE INVENTION

In accordance with one aspect of the invention, embolic particles are provided as defined in the annexed claims that comprise a biodegradable polymer and a therapeutic agent, wherein the particles are configured such that, upon administration to a body lumen of a subject, the therapeutic agent is released from the time of administration up until a first point in time that ranges anywhere from 1 week after administration to 4 weeks after administration, at which point in time the therapeutic agent release ceases. The particles are also configured such that particles remain present in the body lumen from the first point in time at which therapeutic agent release ceases up to a second point in time that ranges anywhere from 2 weeks to about 12 months after the first point in time, at which point the particles are completely degraded.

Other aspects described herein relate to methods of making such particles.

Still other aspects of the invention pertain to injectable compositions that comprise such particles. Also described herein are such injectable compositions for use in methods of treatment.

These and various additional aspects, embodiments and advantages of the present invention will become immediately apparent to those of ordinary skill in the art upon review of the Detailed Description and any appended claims to follow.
The following aspects are preferred embodiments of the description:
1. An embolic particle comprising a biodegradable polymer and a therapeutic agent, wherein the particle is configured such that, upon administration to a body lumen of a subject, the therapeutic agent is released from the time of administration up until a first point in time that ranges anywhere from 1 week after administration to 4 weeks after administration, at which point in time the therapeutic agent release ceases, and such that the particle remains present in the body lumen from the first point in time at which therapeutic agent release ceases up to a second point in time that ranges anywhere from 2 weeks to 12 months after the first point in time, at which point the particle is completely degraded, wherein said embolic particle comprises a biodegradable core and a biodegradable shell, wherein the core contains the biodegradable polymer and the shell contains the biodegradable polymer, wherein said shell comprises apertures but does not comprise said therapeutic agent, and wherein said core comprises said therapeutic agent dispersed throughout and degrades more quickly than said shell, and wherein said polymer is an amino-acid-based poly (ester amide).
2. The embolic particle of aspect 1, wherein said particle ranges between 45 and 300 microns in longest linear cross-sectional dimension.
3. The embolic particle of aspect 1, wherein said particle is spherical or where the particle is a cylindrical particle having an aspect ratio ranging from 2 to 10.
4. The embolic particle of aspect 3, wherein said particle ranges between 45 and 300 microns in diameter.
5. The embolic particle of aspect 1, wherein said amino-acid-based poly(ester amide) comprises an α-amino acid moiety, a diol moiety and a diacid moiety.
6. The embolic particle of aspect 5, wherein said amino-acid-based poly(ester amide) comprises:
   (a) an α-amino acid moiety of the formula, 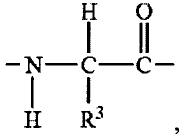 wherein R³ is selected from hydrogen, (C₁-C₆)alkyl, (C₂-C₆)alkenyl, (C₂-C₆)alkynyl, (C₆-C₁₀)aryl(C₁-C₆)alkyl, hydroxy(C₁-C₆)alkyl, hydroxy(C₆-C₁₀)aryl(C₁-C₆)alkyl, carboxy(C₁-C₆)alkyl, carboxy(C₆-C₁₀)aryl(C₁-C₆)alkyl, amino(C₁-C₆)alkyl, 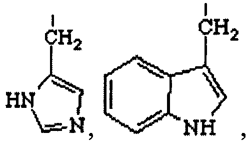 (C₁-C₆)alkyl-amide, and thio(C₁-C₆)alkyl,
   (b) a diol moiety of the formula, -O-R⁴-O-, wherein R⁴ is (C₁-C₂₀)alkyl, and
   (c) a diacid moeity selected from a diacid moiety of the formula, 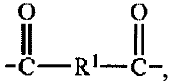 wherein R¹ is (C₁-C₂₀)alkyl, (C₂-C₂₀)alkenyl, or (C₁-C₈)alkyloxy(C₁-C₈)alkyl, an oxo-diacid moiety of the formula, 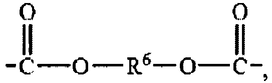 wherin R⁶ is (C₁-C₂₀)alkyl, (C₂-C₂₀)alkenyl, or (C₁-C₈)alkyloxy(C₁-C₈)alkyl, and a combination of both said diacid moiety and said oxo-diacid moiety.
7. The embolic particle of aspect 6, wherein said amino-acid-based poly(ester amide) further comprises an amino-acid moiety of the formula, 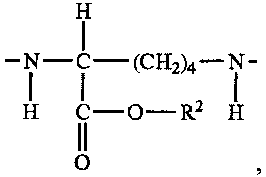 wherein R² is hydrogen, (C₁-C₆)alkyl or (C₆-C₁₀)aryl(C₁-C₆)alkyl.
8. The embolic particle of aspect 7, wherein said amino-acid-based poly(ester amide) comprises a unit of the formula, 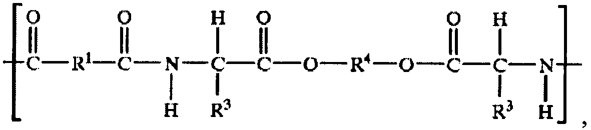 and a unit of the formula, 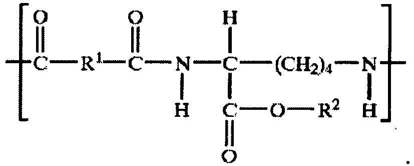
9. The embolic particle of aspect 7, wherein said amino-acid-based poly(ester amide) comprises a unit of the formula, 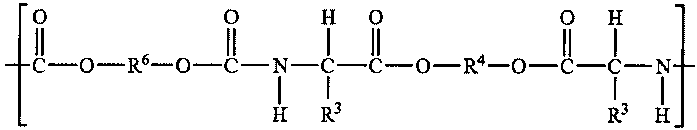 and a unit of the formula, 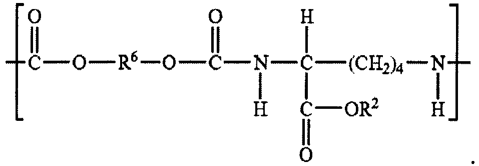
10. The embolic particle of any of aspects 1 or 5 to 9, wherein said amino-acid-based poly(ester amide) is covalently crosslinked.
11. The embolic particle of any of aspects 1-10, wherein said therapeutic agent is an anti-tumor agent.
12. The embolic particle of aspect 11, wherein said anti-tumor agent is paclitaxel.
13. An injectable medical composition comprising particles in accordance with of any of aspects 1-12.
14. The injectable medical composition of aspect 13, comprising a tonicity adjusting agent.
15. The injectable medical composition of any of aspects 13-14, wherein said injectable medical composition is disposed within a glass container or a preloaded medical device.

The injectable medical composition of any of aspects 13-15 for use in a method of embolization comprising injecting the injectable medical composition into a patient is also disclosed.

### BRIEF DESCRIPTION OF THE DRAWINGS

Fig. 1 is a schematic illustration of a spherical core-shell biodegradable particle for reference purposes.
Fig. 2 is a schematic illustration of the particle of Fig. 1 upon degradation of the particle shell.
Fig. 3 is a schematic illustration of a spherical core-shell biodegradable particle in accordance with an embodiment of the present invention.
Fig. 4 is a schematic illustration of the particle of Fig. 3 upon degradation of the particle core.
Fig. 5 is a schematic illustration of a process of producing spherical core-shell biodegradable particles using a triple nozzle apparatus, in accordance with an embodiment of the present invention.
Fig. 6 is a schematic illustration of a process of producing spherical core-shell biodegradable particles using a microfluidic double T-channel apparatus, in accordance with an embodiment of the present invention.

### DETAILED DESCRIPTION

In accordance with various aspects, the invention provides injectable biodegradable polymeric particles that contain a biodegradable polymer and a therapeutic agent.

The particles are configured such that, upon administration to a body lumen (e.g., a blood vessel such as an artery, lymphatic vessel, etc.) of a subject, the therapeutic agent is released from the point of administration up until a first point in time that ranges anywhere from 1 week after administration to 4 weeks after administration, at which point in time the therapeutic agent release ceases. For example, the first point in time where the therapeutic agent release ceases may range anywhere from 1 week to 2 weeks to 3 weeks up to 4 weeks after administration.

The particles arc also configured such that the particles remain in the body lumen for a second period of time after the first period of time has expired (i.e., after therapeutic agent release ceases). For example, the particles may be further configured such that the particles remain present in the body lumen from the first point in time (the time at which therapeutic agent release ceases) up to a second point in time that ranges anywhere from 2 weeks to 12 months after the first point in time, at which point the particles become completely degraded. For example, the second point in time may range anywhere from 2 weeks to 3 weeks to 4 weeks to 2 months to 4 months to 6 months to 8 months to 10 months to 12 months after the first point in time.

As defined herein, the point in time where therapeutic agent ceases is the point where the release rate from the particle(s) drops to below about 5 % of the maximum rate of release.

As defined herein, the point of complete degradation the point where at least 95 wt% degradation of the particle(s) has occurred relative to initial weight of the particle(s) (i.e., the weight at the point of injection).

As used herein a "polymeric particle" is one that contains polymers, typically, from 50 wt% to 75 wt% to 90 wt% to 95 wt% to 97.5 wt% to 99 wt% or more polymers.

As used herein a "biodegradable polymeric particle" is one that undergoes chain cleavage in vivo. As used herein, a polymer is "biodegradable" if it undergoes bond cleavage along the polymer backbone in vivo, regardless of the mechanism of bond cleavage (e.g., enzymatic breakdown, hydrolysis, oxidation, etc.).

As used herein, "polymers" are molecules that contain multiple copies of one or more types of constitutional species, commonly referred to as monomers. The number of monomers within a given polymer may vary widely, ranging, for example, from 5 to 10 to 25 to 50 to 100 to 1000 to 10,000 or more constitutional units. As used herein, the term "monomer" may refer to the free monomers and those that are incorporated into polymers (also referred to herein as monomer "residues"), with the distinction being clear from the context in which the term is used.

As used herein, a "moiety" is a subunit of a polymer and includes monomers and collections of monomers. Moieties may be named herein based on species that are used, or could have been used, to create the moiety (e.g., via a condensation reaction, resulting in the generation of a water molecule, etc.). As a specific example, a "succinic acid moiety", - COCH₂CH₂CO-, may be produced using succinic acid, HOOCCH₂CH₂COOH, via condensation reaction, but can also be produced using another species such as succinyl chloride, ClOCCH₂CH₂COCl.

The injectable particles of the present disclosure may be non-crosslinked or they may be covalently and/or non-covalently crosslinked. Thus, in some embodiments, crosslinking agents such as covalent crosslinking agents or ionic crosslinking agents may be present in the injectable particles, whereas in other embodiments crosslinking agents are absent from the particles. In some embodiments the particles may be crosslinked by exposure to radiation (e.g., gamma or e-beam radiation), which may occur in conjunction with sterilization of particles.

The injectable particles may be used to treat various diseases and conditions in a variety of subjects. Subjects include vertebrate subjects, particularly humans and various warm-blooded animals, including pets and livestock. As used herein, "treatment" refers to the prevention of a disease or condition, the reduction or elimination of symptoms associated with a disease or condition, or the substantial or complete elimination of a disease or condition. Preferred treatments are embolization treatments.

The injectable particles of the invention may vary in shape. In certain embodiments, they are substantially spherical, for example, having the form of a perfect (to the eye) sphere or the form of a near-perfect sphere such as a prolate spheroid (a slightly elongated sphere) or an oblate spheroid (a slightly flattened sphere), among other regular or irregular near-spherical geometries. In embodiments where the particles are substantially spherical, at least half of the particles (50% or more, for example, from 50% to 75% to 90% to 95% or more of a particle sample) may have a sphericity of 0.8 or more (e.g., from 0.80 to 0.85 to 0.9 to 0.95 to 0.97 or more). The sphericity of particles can be determined, for example, using a Beckman Coulter RapidVUE Image Analyzer version 2.06 (Beckman Coulter, Miami, FL). Briefly, the RapidVUE takes an image of continuous-tone (gray-scale) form and converts it to a digital form through the process of sampling and quantization. The system software identifies and measures the particles in an image. The sphericity of a particle, which is computed as Da/Dp (where Da = √(4A/π); Dp = P/π; A = pixel area; P = pixel perimeter), is a value from zero to one, with one representing a perfect circle. A particle is "spherical" if it has a sphericity of 0.8 or more (e.g., from 0.80 to 0.85 to 0.9 to 0.95 to 0.97 or more).

The injectable particles of the present disclosure can vary significantly in size, with typical longest linear cross-sectional dimensions (e.g., the diameter of a sphere, the length of a rod or fiber, etc.) of the particles ranging, for example, from 40 to 5000 microns (µm) (e.g, from 40 to 50 to 100 to 150 to 200 to 250 to 300 to 400 to 500 to 750 to 1000 to 1500 to 2000 to 2500 to 5000 microns), more preferably from 45 to 300 microns. Such particles can be delivered, for example, using a microcatheter (e.g., one having an inside diameter ranging from 530 to 690 microns, among other sizes).

For a collection of particles, the arithmetic mean maximum dimension for the group is preferably within the preceding ranges. The arithmetic mean maximum dimension of a group of particles can be determined using a Beckman Coulter RapidVUE Image Analyzer version 2.06 (Beckman Coulter, Miami, FL), described above. The arithmetic mean maximum dimension of a group of particles (e.g., in a composition) can be determined by dividing the sum of the maximum dimensions (which, for a sphere, is the diameter) of all of the particles in the group by the number of particles in the group.

In certain embodiments, multimodal distributions of particles sizes may be employed. For example, a collection of particles may have a first group of particles with a first arithmetic mean maximum dimension of 40 to 50 microns and a second group of particles having a second arithmetic mean maximum dimension of 100 to 150 microns, among other possibilities.

Biodegradable polymers for use in the embolic particles of the present disclosure include biodegradable polyesters (e.g., polyhydroxy acids), polyorthoesters, polyether esters, polyamides, polyesteramides, polydepsidpetides, polyurethanes, polysaccharides, and polyhydroxyalkanoates, among others. The embolic particles according to the present invention comprise an amino-acid-based poly(ester amide).

Depending on the biodegradation mechanism, a polymer can undergo surface degradation, bulk degradation or a combination of both. Surface versus bulk degradation is often dependent on whether the degradation is via a hydrolytic mechanism (e.g., ester hydrolysis) or via an enzymatic mechanism. In case of degradation by hydrolysis, bulk degradation takes place, but can be controlled by exerting control over the rate of water penetration and material swelling, which are governed by the hydrophilicity of the polymer. In the case of enzyme- or cellular-mediated biodegradation, the mechanism may be mainly via surface degradation. Enzymatic degradation can occur, for example, via hydrolytic or oxidative mechanisms. These degradation mechanisms can occur as a result of the inflammatory foreign body response that occurs upon implantation of the polymeric drug delivery system. Enzymes commonly involved in biodegradation include esterases, proteases, elastases, and peroxidases. See, e.g., Aylvin A. Dias and Marc Hendriks, "Amino Acid-Containing Degradable Polymers & Their Potential in Controlled Drug Delivery," Drug Delivery Technology, May 2010, Vol. 10, No. 4, 20-25.

Biodegradable polymers useful in the present disclosure include those in which the in vivo degradation mechanism is dominated by surface degradation. Where a therapeutic agent is dispersed throughout such a polymer (i.e., where the polymeric material acts as a matrix that entraps the therapeutic agent), the rate of therapeutic agent release from the polymeric material will be controlled by surface degradation as well, allowing drug release to continue until the polymeric material is substantially entirely eroded.

One group of polymers that is used in the embolic particles according to the present invention, and that has demonstrated degradation dominated by surface erosion are the amino-acid-based poly(ester amides) (AA-PEAs). For example, in A. Ghaffar et al., Biomacromolecules 2011,12, 3243-3251, a class of AA-PEAs was subjected to in vitro enzymatic degradation with α-chymotrypsin and proteinase K. The polymers were found to degrade at a steady rate using both enzymes, with a lack of significant changes in the average molecular weight of the remaining polymer, indicating that surface erosion occurred during the enzyme-mediated degradation. No accumulation of acidic byproducts was observed during the course of the experiment. The class of polymers also showed a remarkable hydrolytic stability in the absence of enzymes.

AA-PEAs appear to support a more natural wound healing process than aliphatic polyester-based biomaterials by promoting reendothelialization and lowering inflammatory response. See Kai Guo and C. C. Chu, Journal of Biomedical Materials Research Part B: Applied Biomaterials, Volume 89B, Issue 2, 2008, 491-500. For example, AA-PEAs have shown good tissue and blood compatibility in stent coating applications, with in vivo biocompatibility as tested in porcine coronary arteries showing that the polymer-coated stents had similar injury and inflammation scores to a bare metal stent. See Aylvin A. Dias and Marc Hendriks, Drug Delivery Technology, May 2010, Vol. 10, No. 4, 20-25.

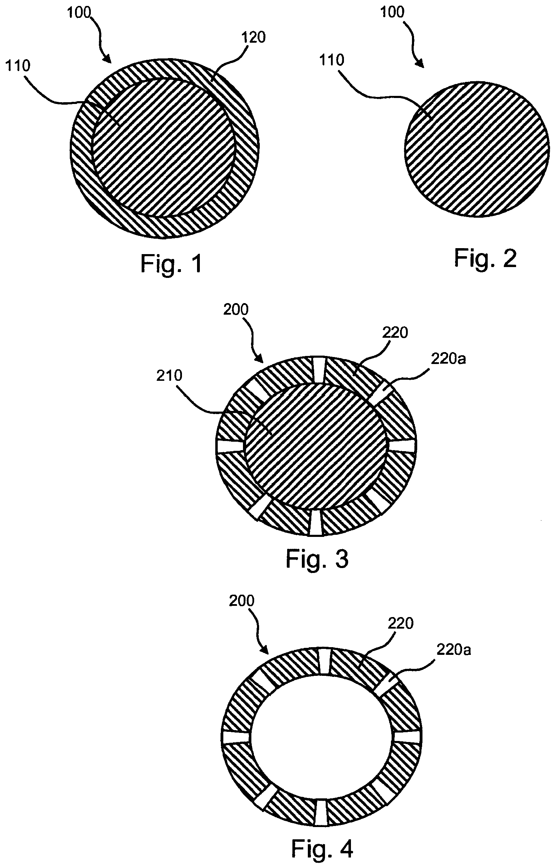

Referring now to Fig. 1, a spherical biodegradable particle 100
is shown, which includes (a) a biodegradable core 110 that contains one or more biodegradable polymers and no therapeutic agent and (b) a biodegradable shell 120 that contains one or more biodegradable polymers and one or more therapeutic agents dispersed throughout.

Referring now to Fig. 3, a substantially spherical
biodegradable particle 200 in accordance with the present disclosure is shown, which includes (a) a biodegradable core 210 that contains one or more biodegradable polymers and one or more therapeutic agents dispersed throughout and (b) a biodegradable shell 220 that contains one or more biodegradable polymers and no therapeutic agent As previously noted, upon administration to a subject, biodegradable particles in accordance with the present disclosure display a first period over which release of one or more therapeutic agents accompanies degradation and a second period during which time degradation of the particles continues, with no accompanying release of therapeutic agent, until complete degradation of the particles is achieved. In a particle 200 like that of Fig. 3, this can be achieved by employing a core 210 that undergoes surface degradation with little to no bulk degradation, with the time for complete degradation of the core 210 corresponding to the first period during which drug release occurs. The shell 220, on the other hand, is formed of a material that degrades more slowly than the core. Moreover, the shell is provided with apertures 22a, which allow diffusion of external species (e.g., water, enzymes, etc.) into the core and which also allow diffusion of internal species (e.g., therapeutic agent and polymer breakdown products) out of the core. In such a system, after the first period (and compete degradation of the core), only the shell 220 remains, as shown schematically in Fig. 4. The shell 220 then continues to degrade without an accompanying release of therapeutic agent until complete degradation of the particle is achieved at the end of the second period.

As noted above, polymers for forming the particles described herein include amino-acid-based poly(ester amides) (AA-PEAs) which comprise an amino acid moiety, preferably an α-amino acid moiety, and additional moieties selected, for example, from polyol moieties (e.g., diol, triol, etc.), polyacid moieties (e.g., diacid, triacid, etc.) and hydroxyacid moieties, among others.

Various AA-PEAs suitable for use in the present disclosure are described in the polymer art and include, for example, those described in the following: Kai Guo and C. C. Chu, "Biodegradable and Injectable Paclitaxel-Loaded Poly(esteramide)s Microspheres: Fabrication and Characterization," Journal of Biomedical Materials Research Part B: Applied Biomaterials, Volume 89B, Issue 2, 2008, 491- 500; M. Vera et al., "Microspheres from new biodegradable poly(ester amide)s with different ratios of L- and D-alanine for controlled drug delivery," Journal of Microencapsulation, September 2006; 23(6): 686- 697; Aylvin A. Dias and Marc Hendriks, "Amino Acid-Containing Degradable Polymers & Their Potential in Controlled Drug Delivery," Drug Delivery Technology, May 2010, Vol. 10, No. 4, 20-25; A. Ghaffar et al., "Monitoring the in Vitro Enzyme-Mediated Degradation of Degradable Poly(ester amide) for Controlled Drug Delivery by LC-ToF-MS," Biomacromolecules 2011,12, 3243-3251; Alfonso Rodriguez-Galan, et al., "Degradable Poly(ester amide)s for Biomedical Applications," Polymers 2011, 3, 65-99; Xuan Pang et al., "Synthesis, characterization and biodegradation of functionalized amino acid-based poly(ester amide)s," Biomaterials 31 (2010) 3745- 3754; U.S. Patent No. 7,304,122 to Chu et al. and U.S. Patent Pub. No. 2004/0063606 to Chu et al.

In certain embodiments, the AA-PEAs of the present disclosure comprise one or more α-amino acid moieties, one or more diol moieties and one or more diacid moieties.

Examples of α-amino acid moieties include moieties of the formula (I), 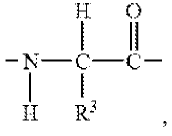
wherein R³ is independently hydrogen (i.e., a glycine moiety), a hydrocarbon group such as (C₁-C₆)alkyl, for example, -CH₃ (i.e., an alanine moiety), - CH(CH₃)CH₃ (i.e., a valine moiety),
-CH(CH₃)CH₂CH₃ (i.e., an isoleucine moiety) and -CH₂CH(CH₃)CH₃ (i.e., a leucine moiety), (C₂-C₆)alkenyl, for example, -CH₂CHCH₂ (i.e., an allylglycine moiety), (C₂-C₆)alkynyl, (C₆-C₁₀)aryl(C₁-C₆)alkyl, for example, -CH₂C₆H₅ (i.e., a phenylalanine moiety), a hydroxyl-substitued hydrocarbon group such as hydroxy(C₁-C₆)alky, for example, -CH₂OH (i.e., a serine moiety) and -CH(OH)CH₃ (i.e., a threonine moiety), and hydroxy(C₆-C₁₀)aryl(C₁-C₆)alkyl, for example, -CH₂C₆H₄OH (i.e., a tyrosine moiety), a carboxy-substitued hydrocarbon group such as carboxy(C₁-C₆)alkyl, for example, CH₂COOH (i.e., an asparatic acid moiety) and
-CH₂CH₂COOH (i.e., a glutamic acid moiety), carboxy(C₁-C₆)alkene and carboxy(C₆-C₁₀)aryl(C₁-C₆)alkyl, and amine-containing hydrocarbon groups such as amino(C₁-C₆)alkyl, for example, -CH₂CH₂CH₂CH₂NH₂ (i.e., a lysine moiety moiety) and -CH₂CH₂NH(NH)NH₂ (i.e., an arginine moiety), or other amine-containing hydrocarbon groups such as 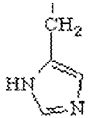 (i.e., histidine moiety) and 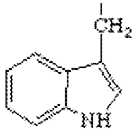 (i.e., tryhptophan moiety), amide-containing hydrocarbon groups such as (C₁-C₆)alkyl-amides, for example, -CH₂CONH₂ (i.e., an asparagine moiety) and -CH₂CH₂CONH₂ (i.e., a glutamine moiety), thio-containing hydrocarbon groups such as thio(C₁-C₆)alkyl, for example, -CH₂SH (i.e., a cysteine moiety) and -CH₂CH₂SCH₃ (i.e., a methionine moiety).

Examples of α-amino acid moieties further include moieties of the formula (II), 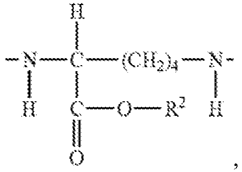 wherein R² is independently hydrogen (i.e., a lysine moiety), (C₁-C₆)alkyl or (C₆-C₁₀)aryl(C₁-C₆)alkyl (e.g., a lysine methyl ester moiety, a lysine ethyl ester moiety, a lysine benzyl ester moiety, etc.).

Examples of diacid moieties include moieties of the formula (III), 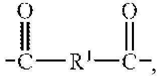 wherein R¹ is independently (C₁-C₂₀)alkyl, for example, methyl, ethyl (i.e., a succinic acid moiety), *n*-propyl, isopropyl, *n*-butyl (i.e., an adipic acid moiety), iso-butyl, *n*-hexyl (i.e., a suberic acid moiety), isohexyl, *n*-octyl (i.e., a sebacic acid moiety), *n*-decyl, *n*-dodecyl, *n-*tetradecyl, etc., (C₂-C₂₀)alkenyl, or (C₁-C₈)alkyloxy(C₁-C₈)alkyl, for example, ethoxyethyl, ethoxy-*n*-butyl, *n*-butoxyethyl, *n*-butoxy-*n*-butyl, etc.

Examples of diacid moieties further include oxo-diacid moieties of the formula (IV), 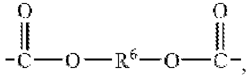 wherin R⁶ is independently (C₁-C₂₀)alkyl, for example, methyl, ethyl, *n*-propyl, isopropyl, *n*-butyl, iso-butyl, *n*-hexyl, isohexyl, *n*-octyl, *n*-decyl, *n*-dodecyl, *n*-tetradecyl, etc., (C₂-C₂₀)alkenyl, or (C₁-C₈)alkyloxy(C₁-C₈)alkyl, for example, ethoxyethyl, ethoxy-*n*-butyl, *n*-butoxyethyl, *n*-butoxy-*n*-butyl, etc.

Examples of diol moieties include moieties of the formula (V), -O-R⁴-O-, wherein R⁴ is independently (C₁-C₂₀)alkyl, for example methyl, ethyl (e.g., a 1,2-ethane diol moiety), *n*-propyl, isopropyl, *n*-butyl (i.e., a 1,4-*n*-butane diol moiety), iso-butyl, *n*-hexyl (i.e., a 1,6-*n*-hexane diol moiety), isohexyl, *n*-octyl (i.e., a 1,8-*n*-octane diol moiety), *n*-decyl (i.e., a 1,10-*n*-decane diol moiety), *n*-dodecyl (i.e., a 1,12-*n*-dodecane diol moiety), *n-*tetradecyl, etc., or (C₁-C₈)alkyloxy(C₁-C₈)alkyl, for example, ethoxyethyl, ethoxy-*n*-butyl, *n-*butoxyethyl, *n*-butoxy-*n*-butyl, etc.

In certain embodiments, AA-PEAs for use herein comprise one or more poly(esteramide) units of the formula (VI), 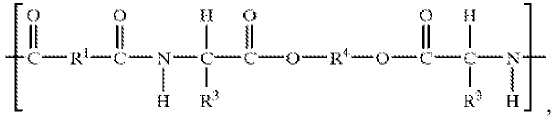 where R¹ is defined above in conjuction with formula (III), where R³ is defined above in conjuction with formula (I) and where R⁴ is defined above in conjuction with formula (V). In certain, more specfic embodiments, R¹ is -(CH₂)ₙ-, where *n* is an integer of 1 or more, for examle, *n*=2, 4, 6, 8, 10 or 12, R³ is selected from isopropyl, isobutyl and benzyl, and R⁴ is -(CH₂)ₙ-, where n is an integer of one or more, for examle, *n*=2, 4, 6, 8, 10 or 12.

In certain embodiments, AA-PEAs for use herein comprise one or more polyamide units of the formula (VII), 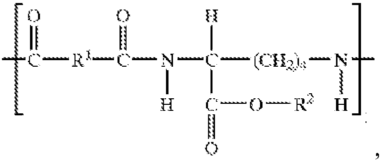 where R¹ is defined above in conjuction with formula (III) and R² is defined above in conjuction with formula (II). In certain more specfic embodiments, R¹ is -(CH₂)ₙ-, where *n* is an integer of one or more, for example, *n*=2, 4, 6, 8, 10 or 12 and R² is selected from hydrogen, methyl, ethyl and benzyl.

In certain embodiments, AA-PEAs for use herein comprise one or more units of the formula (VI) and one or more units of the formula (VII).

In certain embodiments, AA-PEAs for use herein comprise one or more units of the formula (VIII), 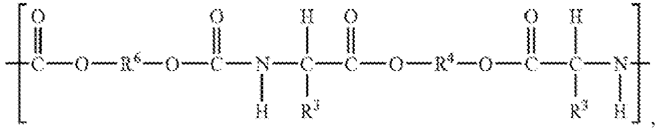 where R³ is defined above in conjuction with formula (I), where R⁴ is defined above in conjuction with formula (V), and where R⁶ is defined above in conjuction with formula (IV). In certain more specfic embodiments, R³ is selected from isopropyl, isobutyl and benzyl, and R⁴ is-(CH₂)ₙ-, where *n* is an integer of one or more, for examle, *n*=2, 4, 6, 8, 10 or 12 and R⁶ is-(CH₂)ₙ- or -(CH₂)ₙ-O-(CH₂)ₙ-, where *n* is an integer of one or more, for examle, *n*=2, 3 4, 6 or 8.

In certain embodiments, AA-PEAs for use herein comprise one or more units of the formula (IX), 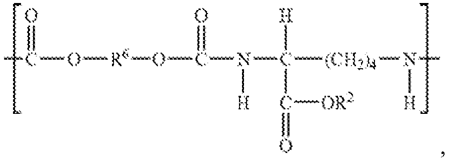 R² is defined above in conjuction with formula (II) and where R⁶ is defined above in conjuction with formula (IV). In certain more specfic embodiments, R² is selected from hydrogen, methyl, ethyl and benzyl and R⁶ is -(CH₂)ₙ- or -(CH₂)ₙ-O-(CH₂)ₙ-, where *n* is an integer of one or more, for examle, *n*=2, 3, 4, 6 or 8.

In certain embodiments, AA-PEAs for use herein comprise one or more units of the formula (VIII) and one or more units of the formula (IX).

"Therapeutic agents," "biologically active agents," "drugs," "pharmaceutically active agents," "pharmaceutically active materials," and other related terms may be used interchangeably herein and include genetic therapeutic agents, non-genetic therapeutic agents and cells. Numerous therapeutic agents can be employed in conjunction with the present disclosure, including those used for the treatment of a wide variety of diseases and conditions (i.e., the prevention of a disease or condition, the reduction or elimination of symptoms associated with a disease or condition, or the substantial or complete elimination of a disease or condition). Numerous therapeutic agents are described here.

Examples of therapeutic agents vary widely and include antioxidants; anti-angiogenic agents; calcium entry blockers (e.g., verapamil, diltiazem, nifedipine); steroidal and non-sterioidal anti-inflammatory agents (e.g., dexamethasone, prednisolone, corticosterone, budesonide, estrogen, acetyl salicylic acid, sulfasalazine, mesalamine, etc.); anesthetic agents (e.g., lidocaine, bupivacaine and ropivacaine); protein kinase and tyrosine kinase inhibitors; anti-proliferative agents; cytostatic agents (i.e., agents that prevent or delay cell division in proliferating cells, for example, by inhibiting replication of DNA or by inhibiting spindle fiber formation) (e.g., toxins, methotrexate, adriamycin, radionuclides, protein kinase inhibitors such as staurosporin and diindoloalkaloids, etc.), agents that inhibit intracellular increase in cell volume (i.e., the tissue volume occupied by a cell) such as cytoskeletal inhibitors (e.g., colchicine, vinblastin, cytochalasins, paclitaxel, etc.) or metabolic inhibitors (e.g., staurosporin, Pseudomonas exotoxin, modified diphtheria and ricin toxins, etc.); trichothecenes (e.g., a verrucarin or roridins); agents acting as an inhibitor that blocks cellular protein synthesis and/or secretion or organization of extracellular matrix (i.e., an "anti-matrix agent" such as colchicine or tamoxifen); various pharmaceutically acceptable salts and derivatives of the foregoing, and combinations of the foregoing, among other agents

Examples of therapeutic agents which may be used in the compositions of the present disclosure thus include toxins (e.g., ricin toxin, radioisotopes, or any other agents able to kill undesirable cells, such as those making up cancers and other tumors such as uterine fibroids) and agents that arrest growth of undesirable cells.

Specific examples of therapeutic agents include anti-tumor agents and may be selected from suitable members of the following: radioisotopes including ⁹⁰Y, ³²P, ¹⁸F, ¹⁴⁰La, ¹⁵³Sm, ¹⁶⁵Dy, ¹⁶⁶Ho, ¹⁶⁹Er, ¹⁶⁹Yb, ¹⁷⁷Lu, ¹⁸⁶Re, ¹⁸⁸Re, ¹⁰³Pd, ¹⁹⁸Au, ¹⁹²Ir, ⁹⁰Sr, ¹¹¹In or ⁶⁷Ga, antineoplastic/antiproliferative/anti-miotic agents including antimetabolites such as folic acid analogs/antagonists (e.g., methotrexate, etc.), purine analogs (e.g., 6-mercaptopurine, thioguanine, cladribine, which is a chlorinated purine nucleoside analog, etc.) and pyrimidine analogs (e.g., cytarabine, fluorouracil, etc.), alkaloids including taxanes (e.g., paclitaxel, docetaxel, etc.), alkylating agents such as alkyl sulfonates, nitrogen mustards (e.g., cyclophosphamide, ifosfamide, etc.), nitrosoureas, ethylenimines and methylmelamines, other aklyating agents (e.g., dacarbazine, etc.), antibiotics and analogs (e.g., daunorubicin, doxorubicin, idarubicin, mitomycin, bleomycins, plicamycin, etc.), platinum complexes (e.g., cisplatin, carboplatin, etc.), antineoplastic enzymes (e.g., asparaginase, etc.), agents affecting microtubule dynamics (e.g., vinblastine, vincristine, colchicine, Epo D, epothilone), caspase activators, proteasome inhibitors, angiogenesis inhibitors (e.g., statins such as endostatin, cerivastatin and angiostatin, squalamine, etc.), rapamycin (sirolimus) and its analogs (e.g., everolimus, tacrolimus, zotarolimus, etc.), etoposides, and many others (e.g., hydroxyurea, flavopiridol, procarbizine, mitoxantrone, campothecin, etc.), various pharmaceutically acceptable salts and derivatives (e.g., esters, etc.) of the foregoing, and combinations of the foregoing, among other agents.

Further therapeutic agents include thrombogenic agents such as homocysteine.

Further therapeutic agents include chemical ablation agents (materials whose inclusion in the formulations of the present disclosure in effective amounts results in necrosis or shrinkage of nearby tissue upon injection) including osmotic-stress-generating agents (e.g., salts, etc.). Specific examples of chemical ablation agents from which suitable agents can be selected include the following: basic agents (e.g., sodium hydroxide, potassium hydroxide, etc.), acidic agents (e.g., acetic acid, formic acid, etc.), enzymes (e.g., collagenase, hyaluronidase, pronase, papain, etc.), free-radical generating agents (e.g., hydrogen peroxide, potassium peroxide, etc.), other oxidizing agents (e.g., sodium hypochlorite, etc.), tissue fixing agents (e.g., formaldehyde, acetaldehyde, glutaraldehyde, etc.), coagulants (e.g., gengpin, etc.), non-steroidal anti-inflammatory drugs, contraceptives (e.g., desogestrel, ethinyl estradiol, ethynodiol, ethynodiol diacetate, gestodene, lynestrenol, levonorgestrel, mestranol, medroxyprogesterone, norethindrone, norethynodrel, norgestimate, norgestrel, etc.), GnRH agonists (e.g, buserelin, cetorelix, decapeptyl, deslorelin, dioxalan derivatives, eulexin, ganirelix, gonadorelin hydrochloride, goserelin, goserelin acetate, histrelin, histrelin acetate, leuprolide, leuprolide acetate, leuprorelin, lutrelin, nafarelin, meterelin, triptorelin, etc.), antiprogestogens (e.g., mifepristone, etc.), selective progesterone receptor modulators (SPRMs) (e.g., asoprisnil, etc.), various pharmaceutically acceptable salts and derivatives of the foregoing, and combinations of the foregoing, among other agents.

The amount of therapeutic agent within the compositions of the present disclosure will vary widely depending on a number of factors, including the disease or condition being treated, the potency of the therapeutic agent, and the volume of particulate composition that is ultimately injected into the subject, among other factors, with the therapeutically effective amount being readily determined by those of ordinary skill in the art. Typical therapeutic agent loadings range, for example, from 0.1 wt% or less, to 0.2 wt% to 0.5 wt% to 1 wt% to 2 wt% to 5 wt% to 10 wt% to 20 wt% or more of the dry weight of the composition.

In certain embodiments, the particles of the present disclosure will optionally include imaging contrast agents in amounts useful to enhance in vivo imaging of the particles. For example, the imaging contrast agents may be provided in particle cores, particle shells, or both. Examples of imaging agents include (a) contrast agents for use in conjunction with magnetic resonance imaging (MRI), including contrast agents that contain elements with relatively large magnetic moment such as Gd(III), Dy(III), Mn(II), Fe(III) and compounds

(including chelates) containing the same, such as gadolinium ion chelated with diethylenetriaminepentaacetic acid, and (b) contrast agents for use in connection with x-ray fluoroscopy, including metals, metal salts and oxides (particularly bismuth salts and oxides), and iodinated compounds, among others.

In certain embodiments, the particles of the present disclosure are rendered magnetic (e.g., they contain magnetized materials) or are rendered susceptible to magnetic fields (e.g., they contain paramagnetic or ferromagnetic materials such as iron). For example, magnetic, paramagnetic or ferromagnetic materials may be provided in particle cores, particle shells, or both. Examples of magnetic, paramagnetic or ferromagnetic materials metals, alloys or compounds (e.g., oxides, etc.) of certain transition, rare earth and actinide elements, preferably, iron or iron oxide. In some embodiments, the magnetic, paramagnetic or ferromagnetic materials are in the form or nanoparticles with typical longest linear cross-sectional dimensions (e.g., the diameter of a sphere, the length of a rod or fiber, etc.) ranging, for example, from 1 to 500 nm (e.g., from 1 to 2 to 5 to 10 to 25 to 50 to 100 to 250 to 500 nm).

Particles suitable for injection can be prepared using any suitable technique. Techniques for forming particles in accordance with the disclosure include those wherein particles are formed from one or more liquid phases (e.g., solutions, suspensions, polymer melts) that contain the polymer of interest and any further ingredients such as solvents, therapeutic agents, imaging contrast agents, magnetic/paramagnetic/ferromagnetic materials, and so forth.

As noted above, and according to the present invention, particles are formed which have a core-shell structure.

Such a structure may be achieved, for example, using one of the technologies explained in K.K. Kim and D.W. Pack (2006) "Microspheres for Drug Delivery," in BioMEMS and Biomedical Nanotechnology Volume 1: Biological and Biomedical Nanotechnology, (M. Ferrari, A.P. Lee and L.J. Lee, Eds.), pp. 19-50, Springer, New York.

In a specific embodiment which is not according to the present invention, but explanatory as to how the particles can be formed, a first core solution comprising dissolved polymer (e.g., AA-PEA), any optional agents (e.g., image contrast agent, magnetic/paramagnetic/ferromagnetic material, etc.) and a suitable solvent (e.g., chloroform, dichloromethane, tetrahydrofuran, etc.) is formed, along with a second shell solution comprising dissolved polymer (e.g., AA-PEA), therapeutic agent (e.g., paclitaxel, etc.), any optional agents (e.g., image contrast agent, magnetic/paramagnetic/ferromagnetic material, etc.) and a suitable solvent (e.g., chloroform, dichloromethane, tetrahydrofuran, etc.). In this embodiment, the core solution may be of a higher viscosity than the shell solution (or vice versa) in order to minimize diffusion of agents (e.g., therapeutic agent, etc.) between the solutions during particle formation. The core and shell may also be formed using immiscible solvents (e.g., polar vs. non-polar solvents) in some embodiments.

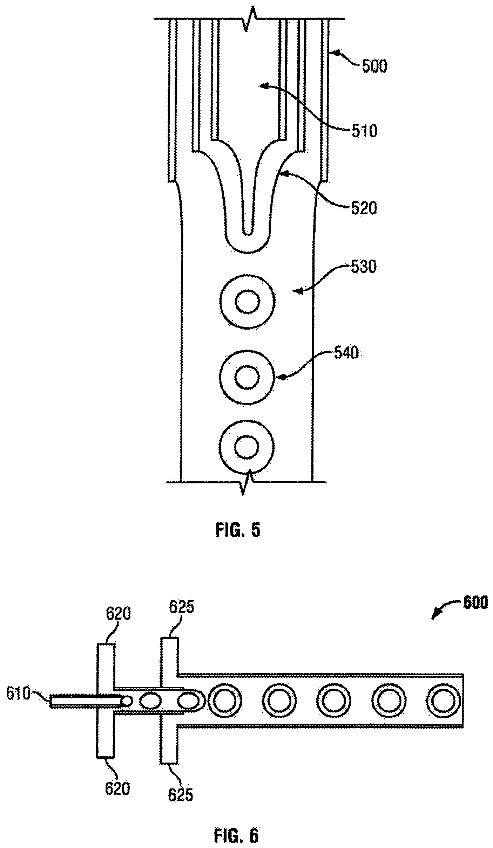

Referring now to Fig. 5, three solutions are simultaneously injected through a triple nozzle apparatus 500. The solutions include the core solution 510, an annular shell solution 520 and an annular, non-solvent carrier stream 530, which allows further control of the droplet size. The non-solvent carrier stream 530 surrounding the coaxial jet formed by core solution 510 and shell solution 520 accelerates the coaxial jet and makes it thinner, eventually causing it to break into particles. More particularly, carrier stream 530 is pumped at a linear velocity greater than that of the polymer streams 510, 520. Thus, frictional contact between the carrier stream 530 and polymer streams 510, 520 generates an additional downward force that effectively pulls the polymer streams 510, 520 away from the tip of the nozzle. The polymer streams 510, 520 are accelerated by this force and, therefore, thinned to a degree depending on the difference in the linear velocities of the carrier stream 530 and polymers streams 510, 520. The carrier stream 530 allows production of core-shell microspheres 540 that are much smaller than the orifice size. The addition of the carrier stream 530 accommodates higher-viscosity materials and reduces the risk of clogging by allowing use of larger nozzles. The core shell particles 540 produced are analogous to those of Fig. 1.

Particles analogous to Fig. 3 and according to the present invention may also be produced by employing first core solution comprising a first dissolved polymer (e.g., a more rapidly degradable AA-PEA), therapeutic agent (e.g., paclitaxel, etc.), any optional agents (e.g., image contrast agent, magnetic/paramagnetic/ferromagnetic material, etc.) and a suitable solvent (e.g., chloroform, tetrahydrofuran, etc.) and a second shell solution comprising a second dissolved polymer (e.g., less rapidly degradable AA-PEA), any optional agents (e.g., image contrast agent, magnetic/paramagnetic/ferromagnetic material, etc.) and a suitable solvent (e.g., chloroform, tetrahydrofuran, etc.). Apertures may be formed, for example, by laser drilling, among other techniques.

In another embodiment, an ultrasonic spray apparatus with a dual feed solution feed may be employed. Such a system is available from Sono-Tek Corporation , Milton, NY, USA.

In another embodiment which is not according to the present invention, but explanatory as to how the particles can be formed, a core comprising polymer (e.g., AA-PEA) and any optional agents (e.g., image contrast agent, magnetic/paramagnetic/ferromagnetic material, etc.) is first formed using a suitable technique, for example, from a first solution comprising polymer and any optional agents using a suitable technique, for example, using an ultrasonic spray technique, a single nozzle droplet generation technique, or a double nozzle technique employing a central polymer solution stream and an annular carrier stream analogous to that described above or an emulsification/solvent evaporation method (see, e.g., M. Vera et al., Journal of Microencapsulation, September 2006; 23(6): 686- 697 and Kai Guo and C. C. Chu, Journal of Biomedical Materials Research Part B: Applied Biomaterials, Volume 89B, Issue 2, 2008, 491-500). Rod shaped (cylindrical) cores may be created, for example, using a technique like that described in W. Engl, et al., "Millifluidic as a versatile reactor to tune size and aspect ratio of large polymerized objects," International Journal of Multiphase Flow 33 (2007) 897-903), whereby such particles are produced in a microfluidic device. In embolic drug eluting particles, a rod (cylindrical) shape may be advantageous over spherical shapes in some embodiments. In this regard, the minimum dimensional cross-section (diameter) of the rod determines the diameter of the vessel to be blocked, whereas the amount of drug being stored is dependent on both the diameter and length of the rod. Consequently, larger volume particles that thus larger drug doses may be allowed to advance into the smaller vessels. Preferred aspect ratios for cylindrical/rod shaped particles (length divided by diameter) range from 2 to 3 to 4 to 5 to 7 to 10 or more.

Once the core is formed, a fluidized bed coating system may be used to apply a suitable shell on the core, for example, using a second solution comprising dissolved polymer, therapeutic agent, any optional agent and a suitable solvent. The thickness of the shell layer depends on the residence time in the fluidized bed. In certain embodiments, two fluid inlets can be employed, one introducing therapeutic agent in solution and another introducing polymer in solution, which would allow one to modify the composition within the shell in a radial direction, for example, an increase or decrease in therapeutic agent content as one proceeds radially from the core.

In certain embodiments, the particles of the present disclosure are stabilized via covalent crosslinking, non-covalent crosslinking, or both. As a specific example of a covalent crosslinking technique, an allyl substituted polymer may be formed (c.g., an AA-PEA having an allylglycine moiety, among many other possibilities). A suitable crosslinking agent is then provided. For example, a molecule having multiple unsaturated groups, for instance, a diacrylate such as polyethylene glycol diacrylate (see Alfonso Rodriguez-Galan, et al., Polymers 2011, 3, 65-99) may be provided. The crosslinking agent may then be introduced during polymer particle formation (e.g., by including a crosslinking agent in a polymer solution used in one of the preceding techniques), with crosslinking subsequently carried out based on a suitable curing mechanism (e.g., using UV irradiation, among other mechanisms).

Regardless of the method of formation, once formed, the particles may then be washed, isolated, sized and lyophilized, as desired.

The particle compositions of the present disclosure may be stored and transported in wet form, for instance, as an aqueous suspension (e.g., AA-PEAs are known which, although enzymatically degradable, demonstrate hydrolytic stability in the absence of enzymes). The particle compositions of the present disclosure may be also be stored and transported in a sterile dry form. In addition to polymer, therapeutic agent and optional contrast agent and optional magnetic/paramagnetic/ferromagnetic material, described above, the wet or dry composition may also optionally contain additional agents, for example, selected from one or more of the following, among others: (a) tonicity adjusting agents such as sugars (e.g., dextrose, lactose, etc.), polyhydric alcohols (e.g., glycerol, propylene glycol, mannitol, sorbitol, etc.) and inorganic salts (e.g., potassium chloride, sodium chloride, etc.), among others, (b) suspension agents including various surfactants, wetting agents, and polymers (e.g., albumen, PEO, polyvinyl alcohol, block copolymers, etc.), among others, and (c) pH adjusting agents including various buffer solutes.

Dry or wet compositions may be shipped, for example, in a syringe, catheter, vial, ampoule, or other container. Dry forms may be mixed with a suitable liquid carrier (e.g. sterile water for injection, physiological saline, phosphate buffer, a solution containing an imaging contrast agent, magnetic/paramagnetic/ferromagnetic material, etc.) prior to administration. In this way the concentration of the composition to be injected may be varied at will, depending on the specific application at hand, as desired by the healthcare practitioner in charge of the procedure. Wet forms (e.g., aqueous suspensions) may also be mixed with a suitable liquid carrier (e.g. sterile water for injection, physiological saline, phosphate buffer, a solution containing contrast agent, magnetic/paramagnetic/ferromagnetic material, etc.) prior to administration, allowing the concentration of administered particles (as well as other optional agents) in the suspension to be reduced prior to injection, if so desired by the healthcare practitioner in charge of the procedure. One or more containers of liquid carrier may also be supplied and shipped, along with the dry or wet particles, in the form of a kit. Kits may also include one or more instruments to assist in delivery such as catheters (e.g., microcatheters), guidewires, endoscopes, hypodermic needles and so forth.

As indicated above, controlled, selective obliteration of the blood supply to tumors is used in treating solid tumors, such as renal carcinoma, bone tumor and liver cancer, among various others. The concept behind this treatment is that preferential blood flow toward a tumor will carry the embolization agent to the tumor thereby blocking the flow of blood which supplies nutrients to the tumor, causing it to shrink. Treatment is enhanced in the present disclosure by including a therapeutic agent (e.g., an anti-tumor agent such as an antineoplastic/antiproliferative/anti-miotic agent, toxin, ablation agent, etc.) in the particulate composition. Embolization may be conducted as an enhancement to chemotherapy or radiation therapy. In other embodiments, the particles may be used to treat benign tumors. For example, fibroids, also known as leiomyoma, leiomyomata or fibromyoma, are the most common benign tumors of the uterus.

The present disclosure also encompasses the claimed particles for use in various methods of administering the particulate compositions of the disclosure to effect embolization. One skilled in the art can determine the most desirable way of administering the particles depending on the type of treatment and the condition of the patient, among other factors. Methods of administration include, for example, percutaneous techniques as well as other effective routes of administration. For example, the particulate compositions of the present disclosure may be delivered through a syringe or through a catheter, for instance, a Tracker® microcatheter (Boston Scientific, Natick, MA, USA), which can be advanced over a guidewire, a steerable microcatheter, or a flow-directed microcatheter (MAGIC, Balt, Montomorency, France).

### EXAMPLES

### Example 1: Preparation of core-shell particles using a microfluidic system (reference example).

A microfluidic double T-channel device is produced using polydimethylsiloxane (PDMS, SYLGARD® 184 SILICONE ELASTOMER KIT, Dow Corning, Midland, Michigan, USA) by a method along the lines described by Brian N. Johnson, "Creation and Application of PDMS Microfluidic Devices," National Nanotechnology Infrastructure Network (NNIN) Lurie Nanofabrication Facility (LNF), University of Michigan, June 11, 2009, 23 pages. The device includes a first T-junction structure with a central inlet channel having a diameter of 10 µm and first side channels having a diameter at the T-junction of 10 µm, and an outlet channel having an outlet diameter of 40 µm, which is fed into a secondary T-junction structure with second side channels having a diameter at the secondary T-junction of 10 µm and an outlet diameter of 130 µm. The cured and removed PDMS structure is sealed to an acrylic plate after applying a plasma oxidation step to both surfaces. The sealed microfluidic device is connected by means of Teflon® tubes to two dual digitally controlled syringe pumps (Fusion 100 Touch, Dual syringe Infusion only pump, KR Analytical Ltd, Sandbach, Cheshire, UK). A first solution of a water soluble poly(ester-amide) (Hybrane H/S80 1700, Polymer Factory Sweden AB, Stockholm, Sweden) is prepared at a concentration of 40 mg/ml. For a second solution, poly (ester-amide) (Hybrane D 2800, Polymer Factory Sweden AB, Stockholm, Sweden) is dissolved in dichloromethane in a nitrogen atmosphere for at least 4 hours to obtain an approximately 40 mg/ml solution of polyester amide. To this solution is added an amount of 10% w/w of paclitaxel (versus weight of dissolved PEA). As illustrated schematically in Fig. 6, the Hybrane H/S80 1700 solution is injected via the central channel 610 of the primary T-junction of the at a rate of 1 ml/hr. The second solution (Hybrane D-paclitaxel) is injected in the two side channels 620 of the primary T-junction at a rate of 2 ml/hr. An aqueous solution (Millipore, deionized) of Tris buffer (pH = 8.5, 0.2 M, Sigma-Aldrich Co., US) containing polyvinyl alcohol (PVA; 1.0 % w/w, MW = 6000, Polysciences Europe GmbH, Germany) is introduced in the two side channels 625 of the secondary T-junction at a rate of 4 ml/hr. The PVA acts as a surfactant to avoid agglomeration of the particles. The liquid including PEA droplets is collected in a flask containing an initial amount of 10 ml of the same aqueous solution as is introduced in the side channels 625. After 30 minutes, the process is stopped and the flask is introduced in a rotary vacuum evaporator (Heidolph Instruments GmbH, Schwabach, Germany) to remove the dichloromethane solvent at reduced pressure (70 mTorr) at room temperature for 30 minutes. After removal of the dichloromethane, solidified microparticles (having PEA cores and PEA/paclitaxel shells) are collected in a 50-mL conical tube (Becton, Dickinson and Company, Franklin Lakes, NJ, USA), centrifuged (1500 rpm, 5 min), and rinsed with deionized water (30 mL) three times to remove excess PVA. The particle suspension is then quick-frozen in liquid nitrogen and lyophilized under reduced pressure to remove the aqueous phase. About 80 mg of particles are obtained per hour, passing inside of the outlet channel with an approximate average speed of 15 cm/s, and approximate size of 43 micrometers and containing approximately 600 beads per second.

### Example 2: Preparation of core-shell particles using an ultrasonic spray system (reference example).

A dual liquid feed spray nozzle (Sono-Tek Corporation, Milton, NY, USA) is mounted vertically 5 cm from the inlet inside of a circular flask (Heidolph Instruments GmbH, Schwabach, Germany). The flask is prefilled 20 ml of an aqueous solution (Millipore, deionized) of Tris buffer (pH = 8.5, 0.2 M, Sigma) containing polyvinyl alcohol (PVA; 1.0 % w/w, MW = 6000, Polysciences GmbH, Germany). The dual feed spray nozzle is connected to two syringe pumps (Sono-Tek, Model 997 syringe pumps) and the nozzle connected to a Precision Ultrasonic Generator (Sono-Tek) set at a frequency of 35 kHz. A first solution (Solution A) of poly (ester-amide) (Hybrane D 2800, Polymer Factory Sweden AB, Stockholm, Sweden) is dissolved in dichloromethane in a nitrogen atmosphere for at least 4 hours to obtain an approximately 10% by weight solution of polyester amide. To the solution is added an amount of 10% w/w of paclitaxel (versus weight of dissolved PEA). A second solution (Solution B) of poly (ester-amide) (Hybrane D 1500, Polymer Factory Sweden AB, Stockholm, Sweden) is dissolved in dichloromethane in a nitrogen atmosphere for at least 4 hours to obtain an approximately 10% by weight solution. Solution A is fed into the outer orifice of the spray nozzle at a rate of 1 ml/min and solution B at the inner inlet at a rate of 0.8 ml/min. The droplet size obtained is approximately 80 µm. After 10 minutes, the process is stopped and the flask is introduced to a rotary vacuum evaporator (Heidolph Instruments GmbH, Schwabach, Germany) to remove the dichloromethane solvent at reduced pressure (70 mTorr) at room temperature for 30 minutes. After removal of dichloromethane, solidified microparticles having a PEA core and a PEA-paclitaxel shell were collected in a 50-mL conical tube (Becton, Dickinson and Company, USA), centrifuged (1500 rpm, 5 min), and rinsed with deionized water (30 mL) three times to remove excess PVA. The particle suspension is then quick-frozen in liquid nitrogen and lyophilized under reduced pressure to remove the continuous aqueous phase.

### Example 3: Preparation of core-shell particles with internalized super-paramagnetic nanoparticles (reference example).

The apparatus of Example 2 is employed, except that the syringe of solution A (PEA + drug) is replaced by a SonicSyringe™ Ultrasonic Dispersion Syringe (Sono-Tek). Magnetic iron oxide Nanocrystals (20 nm) In Water coated with PEG (MKN-IOW-PEG-020) are obtained from MKnano, Mississauga, Canada. The nanoparticle solution is dried at 60°C for 3 days. The dried sample is sonicated for 15 minutes in a sonic bath and then re-dispersed in dichloromethane to a concentration of 0.1 mg/ml. This solution was added to Solution A as used in Example 2 in a ratio of 1:1. Process settings remain similar those of Example 2.

## Claims

1. An embolic particle comprising a biodegradable polymer and a therapeutic agent, wherein the particle is configured such that, upon administration to a body lumen of a subject, the therapeutic agent is released from the time of administration up until a first point in time that ranges anywhere from 1 week after administration to 4 weeks after administration, at which point in time the therapeutic agent release ceases, and such that the particle remains present in the body lumen from the first point in time at which therapeutic agent release ceases up to a second point in time that ranges anywhere from 2 weeks to 12 months after the first point in time, at which point the particle is completely degraded, wherein said embolic particle comprises a biodegradable core and a biodegradable shell, wherein the core contains the biodegradable polymer and the shell contains the biodegradable polymer, wherein said shell comprises apertures but does not comprise said therapeutic agent, wherein said core comprises said therapeutic agent dispersed throughout and degrades more quickly than said shell, and wherein said polymer is an amino-acid-based poly (ester amide).

2. The embolic particle of claim 1, wherein said particle ranges between 45 and 300 microns in longest linear cross-sectional dimension.

3. The embolic particle of claim 1, wherein said particle is spherical or where the particle is a cylindrical particle having an aspect ratio ranging from 2 to 10, and wherein said particle ranges between 45 and 300 microns in diameter.

4. The embolic particle of any of claims 1-3, wherein said amino-acid-based poly(ester amide) comprises an α-amino acid moiety, a diol moiety and a diacid moiety.

5. The embolic particle of claim 4, wherein said amino-acid-based poly(ester amide) comprises:
(a) an α-amino acid moiety of the formula, 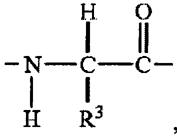 wherein R³ is selected from hydrogen, (C₁-C₆)alkyl, (C₂-C₆)alkenyl, (C₂-C₆)alkynyl, (C₆-C₁₀)aryl(C₁-C₆)alkyl, hydroxy(C₁-C₆)alkyl, hydroxy(C₆-C₁₀)aryl(C₁-C₆)alkyl, carboxy(C₁-C₆)alkyl, carboxy(C₆-C₁₀)aryl(C₁-C₆)alkyl, amino(C₁-C₆)alkyl, 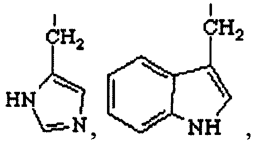 (C₁-C₆)alkyl-amide, and thio(C₁-C₆)alkyl,
(b) a diol moiety of the formula, -O-R⁴-O-, wherein R⁴ is (C₁-C₂₀)alkyl, and
(c) a diacid moeity selected from a diacid moiety of the formula, 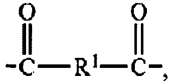 wherein R¹ is (C₁-C₂₀)alkyl, (C₂-C₂₀)alkenyl, or (C₁-C₈)alkyloxy(C₁-C₈)alkyl, an oxo-diacid moiety of the formula, 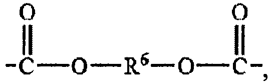 wherin R⁶ is (C₁-C₂₀)alkyl, (C₂-C₂₀)alkenyl, or (C₁-C₈)alkyloxy(C₁-C₈)alkyl, and a combination of both said diacid moiety and said oxo-diacid moiety, and
wherein said amino-acid-based poly(ester amide) optionally further comprises an amino-acid moiety of the formula, 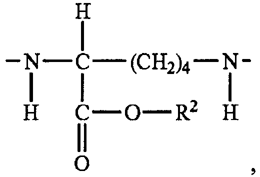 wherein R² is hydrogen, (C₁-C₆)alkyl or (C₆-C₁₀)aryl(C₁-C₆)alkyl.

6. The embolic particle of claim 5, wherein said amino-acid-based poly(ester amide) comprises a unit of the formula, 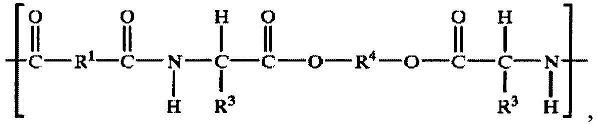 and a unit of the formula,

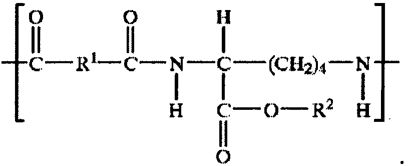

7. The embolic particle of claim 5, wherein said amino-acid-based poly(ester amide) comprises a unit of the formula, 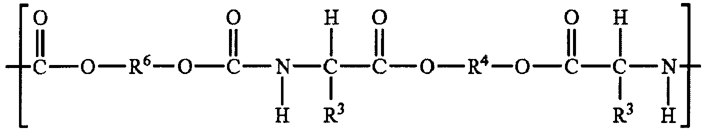 and a unit of the formula,

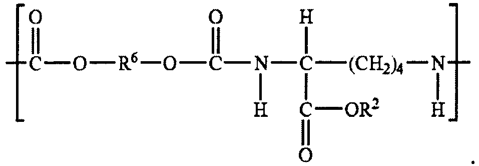

8. The embolic particle of any of claims 4-7, wherein said amino-acid-based poly(ester amide) is covalently crosslinked.

9. The embolic particle of any of claims 1-8, wherein said therapeutic agent is an anti-tumor agent, and wherein said anti-tumor agent preferably is paclitaxel.

10. An injectable medical composition comprising particles in accordance with of any of claims 1-9.

11. The injectable medical composition of claim 10, comprising a tonicity adjusting agent.

12. The injectable medical composition of any of claims 10-11, wherein said injectable medical composition is disposed within a glass container or a preloaded medical device.

## Patentansprüche

1. Embolisches Partikel mit einem biologisch abbaubaren Polymer und einem Therapeutikum, wobei das Partikel derart konfiguriert ist, dass bei Verabreichung an ein Körperlumen eines Subjekts das Therapeutikum vom Zeitpunkt der Verabreichung bis zu einem ersten Zeitpunkt freigesetzt wird, der irgendwo in einem Bereich zwischen einer Woche nach der Verabreichung bis vier Wochen nach der Verabreichung liegt, wobei zu diesem Zeitpunkt die Freisetzung des Therapeutikums endet, und derart, dass das Partikel ab dem ersten Zeitpunkt, zu dem die Freisetzung des Therapeutikums endet, bis zu einem zweiten Zeitpunkt, der irgendwo in einem Bereich zwischen 2 Wochen und 12 Monaten nach dem ersten Zeitpunkt liegt, im Körperlumen verbleibt, wobei das Partikel zu diesem Zeitpunkt vollständig abgebaut ist, wobei das embolische Partikel einen biologisch abbaubaren Kern und eine biologisch abbaubare Hülle aufweist, wobei der Kern das biologisch abbaubare Polymer enthält und die Hülle das biologisch abbaubare Polymer enthält, wobei die Hülle Öffnungen aufweist, aber nicht das Therapeutikum enthält, wobei der Kern das darin verteilte Therapeutikum enthält und schneller abgebaut wird als die Hülle, und wobei das Polymer ein Poly(esteramid) auf Aminosäurebasis ist.

2. Embolisches Partikel nach Anspruch 1, wobei die längste lineare Querschnittsabmessung des Partikels zwischen 45 und 300 µm beträgt.

3. Embolisches Partikel nach Anspruch 1, wobei das Partikel kugelförmig ist, oder wobei das Partikel ein zylinderförmiges Partikel mit einem Aspektverhältnis im Bereich von 2 bis 10 ist, und wobei das Partikel einen Durchmesser im Bereich zwischen 45 und 300 µm hat.

4. Embolisches Partikel nach einem der Ansprüche 1 bis 3, wobei das Poly(esteramid) auf Aminosäurebasis eine α-Aminosäureeinheit, eine Dioleinheit und eine Disäureeinheit aufweist.

5. Embolisches Partikel nach Anspruch 4, wobei das Poly(esteramid) auf Aminosäurebasis aufweist:
(a) eine α-Aminosäureeinheit der Formel 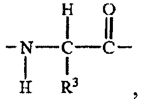 wobei R³ ausgewählt ist aus Wasserstoff, (C₁-C₆)Alkyl, (C₂-C₆)Alkenyl, (C₂-C₆)Alkinyl, (C₆-C₁₀)Aryl(C₁-C₆)Alkyl, Hydroxy(C₁-C₆)Alkyl, Hydroxy(C₆-C₁₀)Aryl(C₁-C₆)Alkyl, Carboxy(C₁-C₆)Alkyl, Carboxy(C₆-C₁₀)Aryl(C₁-C₆)Alkyl, Amino(C₁-C₆)Alkyl, 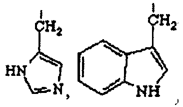 (C₁-C₆)Alkyl-Amid und Thio(C₁-C₆)Alkyl,
(b) eine Dioleinheit der Formel -O-R⁴-O-, wobei R⁴ (C₁-C₂₀)Alkyl ist, und
(c) eine Disäureeinheit, die ausgewählt ist aus einer Disäureeinheit der Formel 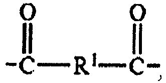 wobei R¹ (C₁-C₂₀)Alkyl, (C₂-C₂₀)Alkenyl oder (C₁-C₈)Alkyloxy(C₁-C₈)Alkyl ist, einer Oxo-Disäureeinheit der Formel 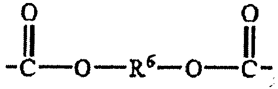 wobei R⁶ (C₁-C₂₀)Alkyl, (C₂-C₂₀)Alkenyl oder (C₁-C₈)Alkyloxy(C₁-C₈)Alkyl ist, und einer Kombination aus der Disäureeinheit und der Oxo-Disäureeinheit, und
wobei das Poly(esteramid) auf Aminosäurebasis optional ferner eine Aminosäureeinheit der Formel 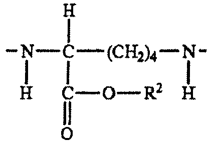 aufweist, wobei R² Wasserstoff, (C₁-C₆)Alkyl oder (C₆-C₁₀)Aryl(C₁-C₆)Alkyl ist.

6. Embolisches Partikel nach Anspruch 5, wobei das Poly(esteramid) auf Aminosäurebasis eine Einheit der Formel 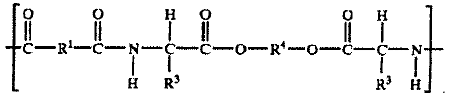 und eine Einheit der Formel 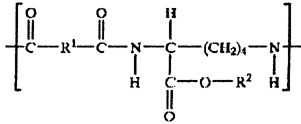 aufweist.

7. Embolisches Partikel nach Anspruch 5, wobei das Poly(esteramid) auf Aminosäurebasis eine Einheit der Formel 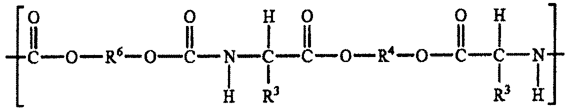 und eine Einheit der Formel 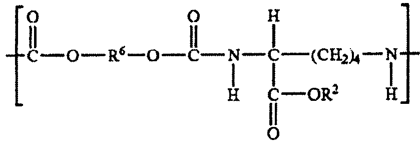 aufweist.

8. Embolisches Partikel nach einem der Ansprüche 4 bis 7, wobei das Poly(esteramid) auf Aminosäurebasis kovalent vernetzt ist.

9. Embolisches Partikel nach einem der Ansprüche 1 bis 8, wobei das Therapeutikum ein Antitumormittel ist, und wobei das Antitumormittel vorzugsweise Paclitaxel ist.

10. Injizierbare medizinische Zusammensetzung mit Partikeln nach einem der Ansprüche 1 bis 9.

11. Injizierbare medizinische Zusammensetzung nach Anspruch 10, mit einem Tonizitätseinstellmittel.

12. Injizierbare medizinische Zusammensetzung nach Anspruch 10 oder 11, wobei die injizierbare medizinische Zusammensetzung in einem Glasbehälter oder in einer vorgeladenen medizinischen Vorrichtung angeordnet ist.

## Revendications

1. Particule embolique comprenant un polymère biodégradable et un agent thérapeutique, où la particule est configurée de telle sorte que, lors de l'administration à une lumière corporelle d'un sujet, l'agent thérapeutique est libéré depuis le temps d'administration jusqu'à un premier point dans le temps qui est situé n'importe quand depuis 1 semaine après l'administration jusqu'à 4 semaines après l'administration, point dans le temps auquel la libération de l'agent thérapeutique cesse, et de telle sorte que la particule demeure présente dans la lumière corporelle depuis le premier point dans le temps auquel la libération de l'agent thérapeutique cesse jusqu'à un second point dans le temps qui est situé n'importe quand depuis 2 semaines jusqu'à 12 mois après le premier point dans le temps, point auquel la particule est complètement dégradée, où ladite particule embolique comprend un noyau biodégradable et une enveloppe biodégradable, où le noyau contient le polymère biodégradable et l'enveloppe contient le polymère biodégradable, où ladite enveloppe comprend des ouvertures mais ne comprend pas ledit agent thérapeutique, où ledit noyau comprend ledit agent thérapeutique dispersé partout et se dégrade plus rapidement que ladite enveloppe, et où ledit polymère est un poly(ester amide) à base d'aminoacide.

2. Particule embolique selon la revendication 1, où ladite particule va entre 45 et 300 micromètres en plus grande dimension en coupe transversale linéaire.

3. Particule embolique selon la revendication 1, où ladite particule est sphérique ou bien où la particule est une particule cylindrique ayant un rapport d'aspect allant de 2 à 10, et où ladite particule va entre 45 et 300 micromètres en diamètre.

4. Particule embolique selon l'une quelconque des revendications 1-3, où ledit poly(ester amide) à base d'aminoacide comprend une entité α-aminoacide, une entité diol et une entité diacide.

5. Particule embolique selon la revendication 4, où ledit poly(ester amide) à base d'aminoacide comprend :
(a) une entité α-aminoacide de formule 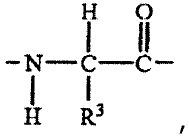 où R³ est choisi parmi l'hydrogène, (C₁-C₆)alkyle, (C₂-C₆)alcényle, (C₂-C₆)alcynyle, (C₆-C₁₀)aryl(C₁-C₆)alkyle, hydroxy(C₁-C₆)alkyle, hydroxy(C₆-C₁₀)aryl(C₁-C₆)alkyle, carboxy(C₁-C₆)alkyle, carboxy(C₆-C₁₀)aryl(C₁-C₆)-alkyle, amino(C₁-C₆)alkyle, 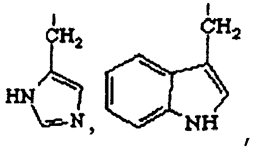 (C₁-C₆)alkyl-amide et thio(C₁-C₆)alkyle,
(b) une entité diol de formule -O-R⁴-O-, où R⁴ est (C₁-C₂₀)alkyle, et
(c) une entité diacide choisie parmi une entité diacide de formule 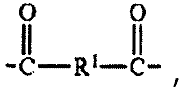 où R¹ est (C₁-C₂₀)alkyle, (C₂-C₂₀)alcényle ou (C₁-C₈)alkyloxy(C₁-C₈)alkyle, une entité oxodiacide de formule 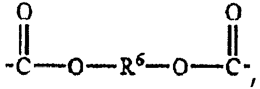 où R⁶ est (C₁-C₂₀)alkyle, (C₂-C₂₀)alcényle ou (C₁-C₈)alkyloxy(C₁-C₈)alkyle, et une combinaison de ladite entité diacide et de ladite entité oxodiacide, et
où ledit poly(ester amide) à base d'aminoacide comprend éventuellement en outre une entité aminoacide de formule, 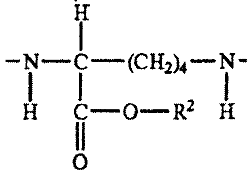 où R² est l'hydrogène, (C₁-C₆)alkyle ou (C₆-C₁₀)aryl(C₁-C₆)alkyle.

6. Particule embolique selon la revendication 5, où ledit poly(ester amide) à base d'aminoacide comprend une unité de formule 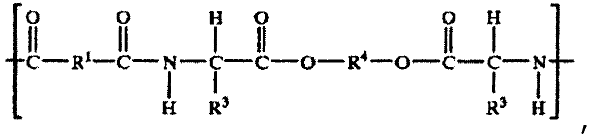 et une unité de formule

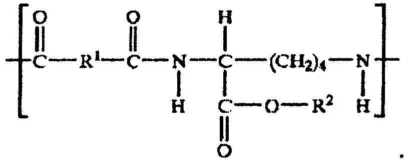

7. Particule embolique selon la revendication 5, où ledit poly(ester amide) à base d'aminoacide comprend une unité de formule 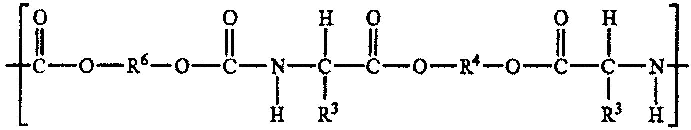 et une unité de formule

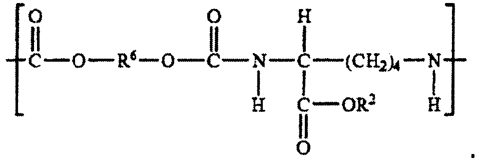

8. Particule embolique selon l'une quelconque des revendications 4-7, où ledit poly(ester amide) à base d'aminoacide est réticulé de manière covalente.

9. Particule embolique selon l'une quelconque des revendications 1-8, où ledit agent thérapeutique est un agent antitumoral, et où ledit agent antitumoral est de préférence le paclitaxel.

10. Composition médicale injectable comprenant des particules selon l'une quelconque des revendications 1-9.

11. Composition médicale injectable selon la revendication 10, comprenant un agent ajustant la pression osmotique.

12. Composition médicale injectable selon l'une quelconque des revendications 10-11, où ladite composition médicale injectable est disposée dans un récipient en verre ou un dispositif médical préchargé.
